# EUROPÄISCHE PATENTSCHRIFT

(11) **EP 4 051 193 B1**
(45) Veröffentlichungstag und Bekanntmachung des Hinweises auf die Patenterteilung: **16.08.2023**
(21) Anmeldenummer: 20799679.4
(22) Anmeldetag: 28.10.2020
(51) Int. Cl.: A61F 9/007

(54) **OPHTHALMOCHIRURGISCHE EINRICHTUNG**
OPHTHALMIC SURGICAL INSTRUMENT
INSTRUMENT CHIRURGICAL OPHTALMIQUE

(30) Priorität: 29.10.2019 DE 102019216669
(43) Veröffentlichungstag der Anmeldung: 07.09.2022
(73) Patentinhaber: Carl Zeiss Meditec AG, 07745 Jena (DE)
(72) Erfinder: KÜBLER, Christoph, 73447 Oberkochen (DE); KOHLHAMMER, Susanne, 89134 Blaustein (DE); NEUMAIER, Markus, 73432 Aalen (DE)
(86) Internationale Anmeldenummer: PCT/EP2020/080248
(87) Internationale Veröffentlichungsnummer: WO 2021/083938

(56) Entgegenhaltungen:
- DE-A1-102015 003 799
- DE-B3-102017 215 677
- US-A1- 2018 318 131

## Beschreibung

Die Erfindung betrifft eine ophthalmochirurgische Einrichtung.

Zur Behandlung einer Augenlinsentrübung, welche in der Medizin als Grauer Star bezeichnet wird, gibt es mehrere chirurgische Techniken. Die am weitesten verbreitete Technik ist die Phakoemulsifikation, bei der eine dünne Hohlnadel (Phakonadel) von einem Handstück in die Augenlinse eingeführt und mit Ultraschall zu Schwingungen angeregt wird. Dabei wird ein Spülfluid (Irrigationsfluid) zugeführt. Die vibrierende Hohlnadel emulsifiziert in ihrer nächsten Umgebung die Augenlinse derart, dass die entstehenden Linsenpartikel durch die Hohlnadel und eine daran angeschlossene Leitung, welche als Aspirationsleitung bezeichnet wird, mittels einer Aspirationspumpe abgesaugt werden können. Ist die Augenlinse vollständig emulsifiziert und entfernt worden, kann in den leeren Kapselsack eine neue künstliche Linse eingesetzt werden, so dass ein derart behandelter Patient wieder ein gutes Sehvermögen erreichen kann.

Die Technik der Phakoemulsifikation funktioniert inzwischen relativ sicher. Ein entsprechendes chirurgisches System wird in Dokument DE102017215677 offenbart.

Es wurde jedoch beobachtet, dass während der Phakoemulsifikation immer wieder ein sehr stark schwankender Augeninnendruck vorliegt, welcher für einen Patienten gefährlich sein kann. Nimmt der Augeninnendruck stark ab, bewegt sich in kurzer Zeit die Hornhaut in Richtung zur Iris, wodurch sich die konvexe Oberfläche der Hornhaut ändert, indem sie uneben, teilweise konkav und gewellt wird. Dies beeinträchtigt die Sicht eines Chirurgen während der Operation, sodass für den Chirurgen unklar ist, wo sich in einem solchen Moment die Spitze einer Phakonadel befindet. Damit steigt das Risiko, dass ungewollt die Phakonadel eine Rückwand des Kapselsackes durchsticht. Dies gilt als eine schwerwiegende Verletzung für einen Patienten, welche nicht mehr von selbst heilt oder durch medizinische Eingriffe geheilt werden kann.

Es gibt mit der heute verfügbaren Technik einige Sicherungsmechanismen, welche eine starke Schwankung eines Augeninnendruckes zu vermeiden versuchen. Es wird jedoch berichtet, dass trotz derartiger Sicherungsmechanismen auch in vermeintlich ungefährlichen Situationen solche Schwankungen des Augeninnendruckes auftreten.

Es besteht eine Aufgabe darin, eine ophthalmochirurgische Einrichtung zu schaffen, mit der bei einer Phakoemulsifikation mit geringem Aufwand wenig oder keine signifikanten Schwankungen eines Augeninnendruckes auftreten.

Die Aufgabe wird durch den Gegenstand des unabhängigen Patentanspruchs 1 gelöst. Vorteilhafte Weiterbildungen der Erfindung sind Gegenstand der Unteransprüche.

Die erfindungsgemäße ophthalmochirurgische Einrichtung weist auf:
- eine Irrigationsfluidleitung, durch welche Irrigationsfluid von einem Irrigationsfluidbehälter zu einem ophthalmochirurgischen Handstück für eine Phakoemulsifikation strömen kann,
- eine erste Fluidpumpe, welche in Strömungsrichtung zwischen dem Irrigationsfluidbehälter und dem Handstück angeordnet ist und eingerichtet ist, Irrigationsfluid zum Handstück zu fördern,
- eine erste Volumenstrom-Bestimmungsvorrichtung, welche in Strömungsrichtung zwischen der ersten Fluidpumpe und dem Handstück angeordnet ist und eingerichtet ist, einen ersten Volumenstrom zu bestimmen,
- eine Aspirationsfluidleitung, durch welche Aspirationsfluid von dem Handstück zu einem Sammelbehälter strömen kann,
- eine zweite Fluidpumpe, welche in Strömungsrichtung zwischen dem Handstück und dem Sammelbehälter angeordnet ist und eingerichtet ist, Aspirationsfluid zum Sammelbehälter zu fördern,
- eine zweite Volumenstrom-Bestimmungsvorrichtung, welche in Strömungsrichtung zwischen dem Handstück und der zweiten Fluidpumpe angeordnet ist und eingerichtet ist, einen zweiten Volumenstrom zu bestimmen,
- ein Differenzelement, welches mit der ersten Volumenstrom-Bestimmungsvorrichtung und der zweiten Volumenstrom-Bestimmungsvorrichtung gekoppelt und eingerichtet ist, eine Differenz aus dem ersten Volumenstrom und dem zweiten Volumenstrom zu bilden, um ein Differenz-Volumenstrom-Signal zu bilden,
- eine Steuerungsvorrichtung mit einem ersten Eingang zum Empfangen des Differenz-Volumenstrom-Signals und einem zweiten Eingang zum Empfangen eines Signals für einen Irrigationsfluid-Solldruck, wobei die Steuerungseinrichtung eingerichtet ist, aus dem Differenz-Volumenstrom-Signal und dem Irrigationsfluid-Solldruck-Signal ein Signal für einen Irrigationsfluid-Steuerdruck zu ermitteln und dieses an einem Ausgang der Steuerungsvorrichtung der ersten Fluidpumpe zuzuführen.

Der Erfindung liegt die Erkenntnis zugrunde, dass eine Schwankung eines Augeninnendruckes nicht nur auftreten kann, wenn eine Okklusion an der Spitze einer Phakonadel aufbricht, sodass sich in der Aspirationsleitung ein relativ hoher Unterdruck in sehr kurzer Zeit normalisiert. Eine Schwankung des Augeninnendruckes kann ebenfalls auftreten, wenn an der Phakonadel keine Okklusion vorliegt, also ganz normal und ohne irgendeine Störung der Operation Linsenpartikel und Fluid abgesaugt werden. Die Erfinder haben beobachtet, dass an der Stelle, an der die Phakonadel durch die Hornhaut gestochen wird, ständig Irrigationsfluid austreten kann. Eine solche Einstichstelle ist größer als der Durchmesser einer Phakonadel, da vor dem Einführen der Phakonadel ein anderes chirurgisches Instrument zur Durchführung einer Kapsulorhexis zum Einsatz kam, welches eine größere Öffnung als die Phakonadel erfordert. Wenn danach die Phakonadel durch diese Öffnung eingeführt wird, ist während einer Phakoemulsifikation ein Auslaufen von Irrigationsfluid an dieser Öffnung unvermeidbar. Ein solcher Verlust von Irrigationsfluid tritt unregelmäßig auf, da ein Chirurg während des Durchführens der Phakoemulsifikation die Phakonadel in der Einstichstelle immer wieder und unregelmäßig bewegt. Der Chirurg achtet während der Phakoemulsifikation darauf, dass das Zertrümmern der harten Augenlinse gelingt. Er kann aber nicht darauf achten, dass durch die Einstichstelle wenig oder gleichmäßig viel Irrigationsfluid austritt, um eine Schwankung des Augeninnendruckes zu vermeiden.

Durch die erfindungsgemäße ophthalmochirurgische Einrichtung wird dieses Problem gelöst. Ein Leck an der Einstichstelle des zu behandelnden Auges und damit der Verlust von Irrigationsfluid lässt sich durch einen Vergleich von einem ersten Volumenstrom in der Irrigationsfluidleitung mit einem zweiten Volumenstrom in der Aspirationsleitung erkennen. Dazu ist eine erste Volumenstrom-Bestimmungsvorrichtung vorgesehen, welche in Strömungsrichtung zwischen der ersten Fluidpumpe und dem Handstück angeordnet ist und eingerichtet ist, einen ersten Volumenstrom zu bestimmen. Ferner ist eine zweite Volumenstrom-Bestimmungsvorrichtung vorgesehen, welche in Strömungsrichtung zwischen dem Handstück und der zweiten Fluidpumpe angeordnet ist und eingerichtet ist, einen zweiten Volumenstrom zu bestimmen. Ein Differenzelement, welches mit der ersten Volumenstrom-Bestimmungsvorrichtung und der zweiten Volumenstrom-Bestimmungsvorrichtung gekoppelt ist, ist eingerichtet, eine Differenz aus dem ersten Volumenstrom und dem zweiten Volumenstrom zu bilden, um ein Differenz-Volumenstrom-Signal zu bilden.

Wenn die Differenz aus dem ersten Volumenstrom und dem zweiten Volumenstrom null beträgt, liegt kein Leck an der Einstichstelle des Auges vor. Wenn die Differenz jedoch größer null ist, liegt ein Leck an der Öffnung vor, durch welche die Phakonadel die Hornhaut durchdringt. Dieses Differenz-Volumenstrom-Signal kann gemäß der Erfindung einem ersten Eingang einer Steuerungsvorrichtung zugeführt werden, welche eingerichtet ist, an einem zweiten Eingang ein Signal für einen Irrigationsfluid-Solldruck zu empfangen. Die Steuerungsvorrichtung verarbeitet das Differenz-Volumenstrom-Signal und das Signal für den Irrigationsfluid-Solldruck derart, dass ein Signal für einen Irrigationsfluid-Steuerdruck an einem Ausgang der Steuerungsvorrichtung bereitgestellt wird, um dieses der ersten Fluidpumpe zuzuführen. Durch die erste Fluidpumpe wird dann der Druck des Irrigationsfluids in der Irrigationsfluidleitung so geändert, dass der Verlust an Irrigationsfluid an der Öffnung der Hornhaut kompensiert werden kann, wodurch eine Schwankung des Augeninnendruckes gering ausfällt oder gar nicht auftritt. Der Aufwand für den Einsatz einer ersten Volumenstrom-Bestimmungsvorrichtung und einer zweiten Volumenstrom-Bestimmungsvorrichtung sowie die Verarbeitung eines daraus abgeleiteten Differenzsignals und ein entsprechendes Ansteuern der ersten Fluidpumpe ist gering. Da während der Phakoemulsifikation der normale Betrieb ohne Okklusion an der Phakonadel einen großen Anteil der aufgewendeten Zeit ausmacht, lässt sich mittels der erfindungsgemäßen Einrichtung während dieser Zeit eine Schwankung des Augeninnendruckes klein halten oder vermeiden, was eine deutliche Verbesserung im Vergleich zu herkömmlichen Einrichtungen zur Phakoemulsifikation darstellt.

Die Steuerungsvorrichtung weist bevorzugt ein Multiplikationselement auf, welches eingerichtet ist, an einem Eingang das Differenz-Volumenstrom-Signal zu empfangen und an einem Ausgang ein Signal für einen Differenzdruck auszugeben. Das Multiplikationselement wandelt somit das Differenz-Volumenstrom-Signal zu einem Drucksignal um. Zusätzlich weist die Steuerungsvorrichtung ein Addierelement auf, welches eingerichtet ist, an einem ersten Eingang ein Signal für den Irrigationsfluid-Solldruck zu empfangen, und an einem zweiten Eingang das Signal für den Differenzdruck zu empfangen, und an einem Ausgang ein Signal für den Irrigationsfluid-Steuerdruck dem Ausgang der Steuerungseinrichtung zuzuführen. Das Addierelement verarbeitet somit zwei Druckwerte und addiert diese. Das Signal für den Irrigations-Solldruck kann von einer Eingabevorrichtung wie zum Beispiel einem Fußpedal zugeführt werden. Die Summe der beiden Drücke kann daraufhin als Steuerdruck der ersten Fluidpumpe in der Irrigationsfluidleitung zugeführt werden.

Gemäß einer Ausführungsform weist die ophthalmochirurgische Einrichtung ein erstes Zeitglied auf, welches eingerichtet ist, den Verlauf des Irrigationsfluid-Solldruckes in Abhängigkeit von der Zeit vorzugeben, wobei das erste Zeitglied mit dem zweiten Eingang der Steuerungsvorrichtung gekoppelt ist. Der Betrag eines Irrigationsfluid-Solldruckes muss somit nicht abrupt durch Einsatz eines Ein/Aus-Schalters der Steuerungsvorrichtung zugeführt werden, sondern kann aufgrund des ersten Zeitgliedes zum Beispiel gemäß einer Exponential-Funktion oder einer Funktion höherer Ordnung langsam auf den Betrag des Irrigationsfluid-Solldruckes ansteigen. Dadurch werden zu schnelle Druckveränderungen in der Irrigationsfluidleitung vermieden, welche sich in der Irrigationsfluidleitung bis zum Handstück und dem Auge fortpflanzen würden und eine Instabilität des Auges verursachen könnten.

Bevorzugt ist eine erste Zeitkonstante des ersten Zeitgliedes einstellbar. In Abhängigkeit von der Art der ersten Fluidpumpe, der Länge der Irrigationsfluidleitung, dem Querschnitt der Irrigationsfluidleitung und der Beschaffenheit der Innenwand der Irrigationsfluidleitung kann mittels der einstellbaren Zeitkonstante des ersten Zeitgliedes zum einen eine schnelle Änderung des Druckes in der Irrigationsfluidleitung erreicht werden. Wenn die Zeitkonstante einstellbar ist, kann zum anderen ein Einschaltvorgang und ein Ausschaltvorgang so gesteuert werden, dass ein Überschwingen eines Signals vermieden wird. Es ist dadurch möglich, einen Endwert nicht durch eine Sprungfunktion, sondern mit einer zeitlichen Verzögerung zu erreichen, wobei dies trotzdem noch schnell erfolgt. Dies ist aus medizinischer Sicht vorteilhaft, da ein Auge während einer Operation somit keinen stoßartigen Belastungen ausgesetzt ist, sodass geometrische Bewegungen des Auges gedämpft erfolgen.

Gemäß einer weiteren Ausführungsform ist ein zweites Zeitglied vorgesehen, welches eingerichtet ist, den Verlauf des Aspirationsfluid-Solldruckes in Abhängigkeit von der Zeit vorzugeben und der zweiten Fluidpumpe zuzuführen. Besonders bevorzugt ist eine zweite Zeitkonstante des zweiten Zeitgliedes einstellbar. Damit kann der Solldruck in der Aspirationsleitung zum Beispiel gemäß einer Exponential-Funktion oder einer Funktion höherer Ordnung langsam auf den Betrag des Aspirationsfluid-Solldruckes ansteigen. Dadurch wird erreicht, dass allein durch das Ansteuern der zweiten Fluidpumpe keine zusätzlichen Druckschwankungen in die Aspirationsleitung eingebracht werden. Besonders bevorzugt weist die ophthalmochirurgische Einrichtung sowohl das erste Zeitglied als auch das zweite Zeitglied auf, deren jeweilige Zeitkonstanten einstellbar sind. Damit kann je nach Länge der Irrigationsfluidleitung bis zum Handstück und ihrer Beschaffenheit sowie der Aspirationsleitung und ihrer Beschaffenheit das Ansteigen eines Solldruckes in der Irrigationsfluidleitung und in der Aspirationsleitung so eingestellt werden, dass während der Phakoemulsifikation bei sich änderndem Betrag des durch ein Leck an der Hornhaut abhanden kommenden Irrigationsfluides der Augeninnendruck nahezu oder vollständig konstant bleibt.

Die erste Zeitkonstante kann in einem Bereich von 50 Millisekunden bis 1000 Millisekunden liegen, und die zweite Zeitkonstante kann in einem Bereich von 50 Millisekunden bis 1000 Millisekunden liegen. Ein nahezu oder vollständig stabiler Augeninnendruck lässt sich besonders zuverlässig erreichen, wenn eine Differenz zwischen der ersten Zeitkonstante und der zweiten Zeitkonstante einen Betrag aufweist, welcher in einem Bereich von größer als null und kleiner als 200 Millisekunden liegt.

Weitere Vorteile und Merkmale der Erfindung werden mit Bezug auf die nachfolgenden Zeichnungen erklärt, in welchen zeigen:
- Fig. 1: eine Ausführungsform einer ophthalmochirurgischen Einrichtung, und
- Fig. 2: schematische Diagramme von Volumenstromverläufen und Druckverläufen in Abhängigkeit von der Zeit bei einem Einsatz der ophthalmochirurgischen Einrichtung

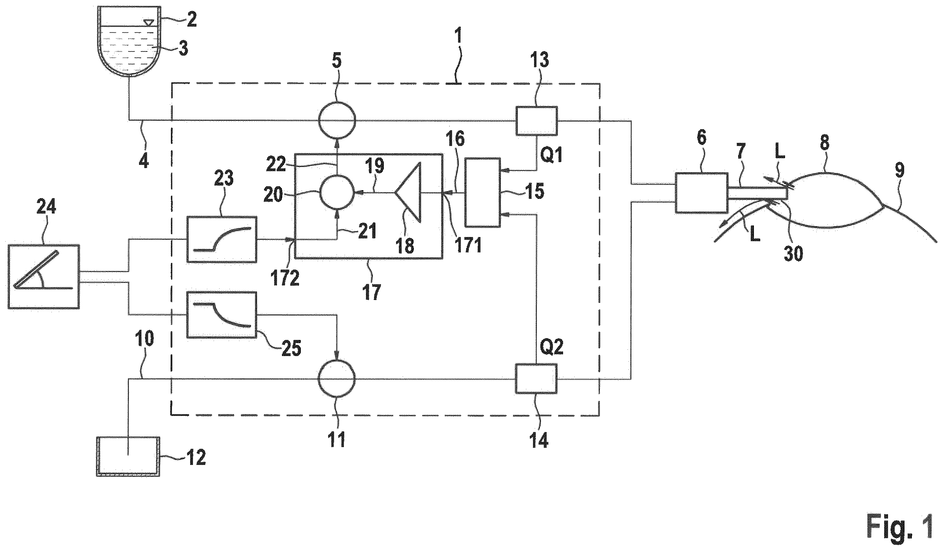

Fig.1 zeigt in einer schematischen Darstellung eine Ausführungsform einer ophthalmochirurgischen Einrichtung 1. In einem Irrigationsfluidbehälter 2 ist ein Irrigationsfluid 3 enthalten, welches durch eine daran angeschlossene Irrigationsfluidleitung 4 zu einer ersten Fluidpumpe 5, welche eine Membranpumpe sein kann, geführt wird. Von dort kann das Irrigationsfluid 3 entlang der Irrigationsfluidleitung 4 bis zu einem Handstück 6 für die Phakoemulsifikation strömen und durch eine Phakonadel 7, welche durch eine Hornhaut 8 gestochen ist, zu einer zu behandelnden Augenlinse eines Auges 9 gelangen. Bei Betrieb einer zweiten Fluidpumpe 11, welche eine Saugpumpe wie zum Beispiel eine Membranpumpe ist, können durch eine Leitung innerhalb der Phakonadel 7 und des Handstückes 6 emulsifizierte Linsenpartikel und Fluid entlang einer Aspirationsfluidleitung 10 zur zweiten Fluidpumpe 11 gelangen. Von der zweiten Fluidpumpe 11 werden die Linsenpartikel und das Fluid, also insgesamt das Aspirationsfluid, entlang der Aspirationsfluidleitung 10 zu einem Sammelbehälter 12 befördert.

In Strömungsrichtung zwischen der ersten Fluidpumpe 5 und dem Handstück 6 ist eine erste Volumenstrom-Bestimmungsvorrichtung 13 angeordnet, mit welcher ein erster Volumenstrom Q1 in der Irrigationsfluidleitung 4 bestimmt werden kann. Bevorzugt wird der erste Volumenstrom Q1 durch die Volumenstrom-Bestimmungsvorrichtung 13 indirekt bestimmt, indem zum Beispiel eine Position einer Membran oder eines Schwimmers erfasst und daraus der Volumenstrom ermittelt wird. In Strömungsrichtung zwischen dem Handstück 6 und der zweiten Fluidpumpe 11 ist eine zweite Volumenstrom-Bestimmungsvorrichtung 14 angeordnet, mit welcher ein zweiter Volumenstrom Q2 in der Aspirationsleitung 10 bestimmt werden kann. Das Signal des ersten Volumenstroms Q1 und das Signal des zweiten Volumenstroms Q2 werden zu einem Differenzelement 15 geleitet, welches eingerichtet ist, eine Differenz aus dem ersten Volumenstrom Q1 und dem zweiten Volumenstrom Q2 zu bilden, um ein Signal 16 entsprechend einem Differenz-Volumenstrom DQ zu bilden. Das Signal 16 ist somit die Differenz von Q1 minus Q2.

Dieses Signal 16 wird einem ersten Eingang 171 einer Steuerungsvorrichtung 17 zugeführt und gelangt dort zu einem Multiplikationselement 18, welches eingerichtet ist, an einem Eingang das Signal 16 zu empfangen und an einem Ausgang ein Signal 19 für einen Differenzdruck auszugeben. Die Steuerungseinrichtung 17 weist zusätzlich ein Addierelement 20 auf, welches eingerichtet ist, an einem ersten Eingang ein Signal 21 für den Irrigationsfluid-Solldruck zu empfangen, und an einem zweiten Eingang das Signal 19 für den Differenzdruck zu empfangen. Das Addierelement 20 verarbeitet diese beiden Signale 19 und 21 derart, dass die Summe daraus gebildet wird, um an einem Ausgang des Addierelementes 20 ein Signal 22 für einen Irrigationsfluid-Steuerdruck dem Ausgang der Steuerungseinrichtung 17 zuzuführen. Das Signal 22 für den Irrigationsfluid-Steuerdruck wird dann der ersten Fluidpumpe 5 zugeführt, welche den Druck des Irrigationsfluides 3 in der Irrigationsfluidleitung 4 entsprechend ändert.

Wenn die Differenz aus dem ersten Volumenstrom und dem zweiten Volumenstrom null beträgt, ist das Signal 22 für den Irrigationsfluid-Steuerdruck ebenfalls null und die erste Fluidpumpe 5 erfährt keine Änderung in ihrer Ansteuerung. Wenn das Signal 16 aber ungleich null und üblicherweise größer als null ist, erfährt die erste Fluidpumpe 5 durch das Signal 22 eine Änderung in ihrer Ansteuerung, sodass sie den Druck für das Irrigationsfluid 3 in der Irrigationsfluidleitung 4 ändert. Damit wird dem ungewollten Abfließen von Fluid aus dem Auge 9 aus einem Leck 30 entgegengewirkt, siehe mit L bezeichnete Pfeile in Fig. 1.

Die ophthalmochirurgische Einrichtung 1 weist zusätzlich ein erstes Zeitglied 23 auf, welches eingerichtet ist, den Verlauf des Irrigationsfluid-Solldruckes in Abhängigkeit von der Zeit vorzugeben. Das erste Zeitglied 23 ist mit einem zweiten Eingang 172 der Steuerungsvorrichtung 17 gekoppelt, woraufhin das Signal 21 für den Irrigationsfluid-Solldruck dem Addierelement 20 zugeführt wird. Das erste Zeitglied 23 ist mit einem Fußpedal 24 gekoppelt, das bei einer entsprechenden Betätigung durch einen Operateur ein Signal für den Irrigationsfluid-Solldruck erhält.

Das Fußpedal 24 ist zusätzlich mit einem zweiten Zeitglied 25 gekoppelt, welchem bei zugehöriger Betätigung des Fußpedals 24 ein Signal für einen Aspirationsfluid-Solldruck zugeführt werden kann. Das zweite Zeitglied 25 ist eingerichtet, den Verlauf des Aspirationsfluid-Solldruckes in Abhängigkeit von der Zeit vorzugeben, welcher dann der zweiten Fluidpumpe 19 zugeführt werden kann.

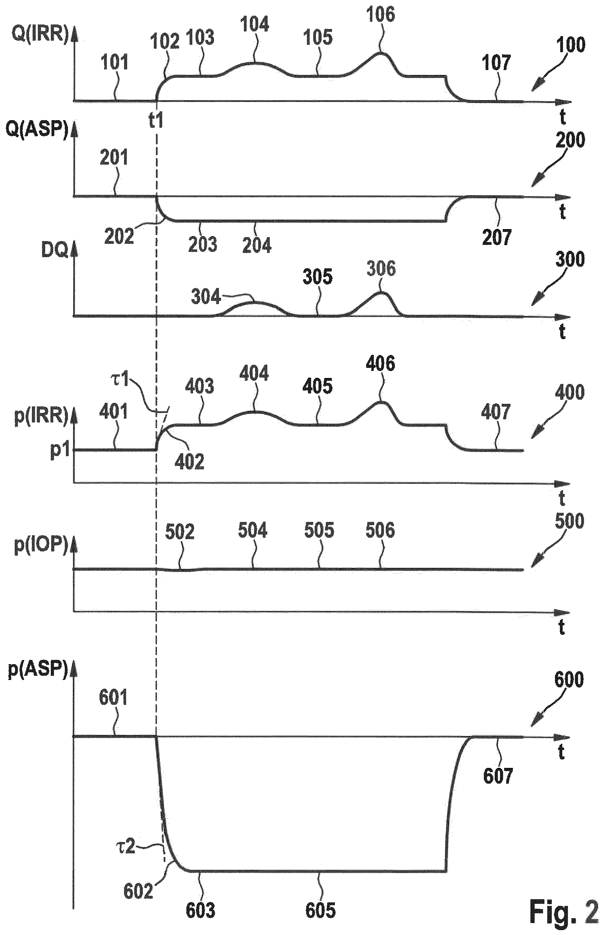

Figur 2 zeigt mehrere schematische Diagramme von Volumenstromverläufen und Druckverläufen in Abhängigkeit von der Zeit.

Diagramm 100 zeigt einen Verlauf eines Irrigationsfluid-Volumenstroms Q(IRR) in Abhängigkeit von der Zeit. Im Zeitraum bis zum Zeitpunkt t1 fließt noch kein Irrigationsfluid durch die Irrigationsfluidleitung, siehe 101 in Diagramm 100. Dies gilt auch für das Aspirationsfluid in der Aspirationsfluidleitung, siehe 201 in Diagramm 200, welches den Verlauf des Aspirationsfluid-Volumenstroms Q(ASP) in Abhängigkeit von der Zeit darstellt. In der Irrigationsfluidleitung herrscht bis zum Zeitpunkt t1 ein stationärer Druck p1, siehe 401 in Diagramm 400, welches den Verlauf des Irrigationsfluid-Druckes p(lRR) in Abhängigkeit von der Zeit darstellt. In der Aspirationsfluidleitung ist bis zum Zeitpunkt t1 der Aspirationsdruck gleich null, siehe 601 in dem Diagramm 600, welches den Aspirationsfluiddruck p(ASP) in Abhängigkeit von der Zeit darstellt. Der Augeninnendruck p(IOP) in Abhängigkeit von der Zeit ist in Diagramm 500 dargestellt. Daraus ist ersichtlich, dass bis zum Zeitpunkt t1 der Augeninnendruck größer als null ist, was dem normalen Zustand des Auges entspricht.

Es wird angenommen, dass zum Zeitpunkt t1 das Fußpedal betätigt wird, sodass die erste Fluidpumpe 5 und die zweite Fluidpumpe 11 jeweils Fluid pumpen. Der Irrigationsfluid-Volumenstrom steigt an, siehe 102, und erreicht einen stationären Wert, siehe 103 in Diagramm 100. Gleichzeitig steigt auch der Aspirationsfluid-Volumenstrom an, siehe 202, und erreicht ebenfalls einen stationären Wert, siehe 203 in Diagramm 200. Da Irrigationsfluid in der Irrigationsfluidleitung und Aspirationsfluid in der Aspirationsleitung strömen, baut sich in diesen Leitungen auch ein zugehöriger Fluiddruck auf. Der Irrigationsfluiddruck p(IRR) steigt an, siehe 402, und erreicht einen stationären Wert, siehe 403 in Diagramm 400. In gleicher Weise steigt der Aspirationsfluiddruck an, siehe 602, und erreicht einen stationären Wert, siehe 603 in Diagramm 600. Wenn sich die erste Zeitkonstante τ1 für den Anstieg des Irrigationsfluiddruckes und die zweite Zeitkonstante τ2 für den Anstieg des Aspirationsfluiddruckes um nicht mehr als 200 Millisekunden unterscheiden, kommt es nur zu einem geringen oder gar keinem Abfall des Augeninnendruckes, siehe 502 in Diagramm 500.

Tritt nun ein Verlust an Irrigationsfluid auf, da ein Leck 30 am Auge vorhanden ist, sinkt der Irrigationsfluiddruck in der Irrigationsfluidleitung 4 zwischen der ersten Pumpe 5 und der Phakonadel 7 ab, sodass die erste Pumpe 5 einen größeren Irrigationsfluid-Volumenstrom bereitstellt, siehe 104. Der Aspirationsfluid-Volumenstrom ist davon unbeeinflusst und bleibt konstant, siehe 204. Durch die erfindungsgemäße ophthalmochirurgische Einrichtung 1 wird eine Differenz zwischen dem Irrigationsfluid-Volumenstrom und dem Aspirationsfluid-Volumenstrom ermittelt, welche größer als null ist, siehe 304 in Diagramm 300, welches einen Differenz-Volumenstrom DQ in Abhängigkeit von der Zeit darstellt. Damit wird von dem Addierelement 20 ein Signal des Irrigationsfluid-Solldruckes und ein Signal eines Differenzdruckes empfangen, sodass auf die erste Fluidpumpe 5 ein entsprechend erhöhter Irrigationsfluid-Steuerdruck wirkt, siehe 404. Während Irrigationsfluid aus einem Leck 30 am Auge 9 austritt, herrscht somit ein höherer Irrigationsfluiddruck in der Irrigationsfluidleitung. Dies hat zur Folge, dass während dieser Zeit im Auge der Augeninnendruck unverändert bleibt, siehe 504.

Wenn die Größe des Lecks 30 wieder abnimmt, zum Beispiel aufgrund einer anderen Lage der Phakonadel, und somit auch ein ungewolltes Abfließen von Irrigationsfluid am Auge abnimmt, kann die erste Fluidpumpe mit einem geringeren Volumenstrom, siehe 105, den üblichen Irrigationsfluiddruck aufrechterhalten. Somit reduziert sich der Differenz-Volumenstrom, siehe 305. Der Irrigationsfluiddruck erreicht damit wieder den Solldruck, siehe 405, der dem Druck gemäß 403 entspricht. Der Augeninnendruck bleibt dabei unverändert, siehe 505. Der Druck in der Aspirationsfluidleitung ist davon unbeeinflusst, siehe 605.

Die Schwankung des Irrigationsfluid-Volumenstroms kann auch relativ hoch sein, siehe 106, wodurch ein höherer Betrag für den Differenz-Volumenstrom DQ erfasst wird, siehe 306. Dies führt zu einer höheren Änderung des Irrigationsfluiddruckes, siehe 406, sodass der Augeninnendruck unverändert bleiben kann, siehe 506.

Wenn das Fußpedal nicht mehr betätigt wird, erfolgt keine Ansteuerung des ersten Pumpe 5 und der zweiten Pumpe 11, sodass der Irrigations-Volumenstrom und der Aspirations-Volumenstrom auf den Betrag null absinken, siehe 107 und 207. Dann sinken auch der Druck in der Irrigationsfluidleitung und in der Aspirationsleitung ab, siehe 407 und 607.

### Bezugszeichen:

- 1: Ophthalmochirurgische Einrichtung
- 2: Irrigationsfluidbehälter
- 3: Irrigationsfluid
- 4: Irrigationsfluidleitung
- 5: Erste Fluidpumpe
- 6: Ophthalmochirurgisches Handstück
- 7: Phakonadel
- 8: Hornhaut
- 9: Auge
- 10: Aspirationsfluidleitung
- 11: zweite Pumpe
- 12: Sammelbehälter
- 13: erste Volumenstrom-Bestimmungsvorrichtung
- 14: zweite Volumenstrom-Bestimmungsvorrichtung
- 15: Differenzelement
- 16: Differenz-Volumenstrom-Signal
- 17: Steuerungseinrichtung
- 18: Multiplikationselement
- 19: Differenzdruck-Signal
- 20: Addierelement
- 21: Irrigationsfluid-Solldruck-Signal
- 22: Irrigationsfluid-Steuerdruck-Signal
- 23: Erstes Zeitelement
- 24: Fußpedal
- 25: zweites Zeitelement
- 171: erster Eingang der Steuerungseinrichtung
- 172: zweiter Eingang der Steuerungseinrichtung
- DQ: Differenz-Volumenstrom
- L: Leck
- p: Druck
- p(ASP): Aspirationsfluiddruck
- p(IOP): Augeninnendruck
- p(IRR): Irrigationsfluiddruck
- Q1: erster Volumenstrom
- Q2: zweiter Volumenstrom
- Q(ASP): Aspirationsfluid-Volumenstrom
- Q(IRR): Irrigationsfluid-Volumenstrom
- t: Zeit
- τ: Zeitkonstante

## Patentansprüche

1. Ophthalmochirurgische Einrichtung (1), aufweisend:
- eine Irrigationsfluidleitung (4), durch welche Irrigationsfluid (3) von einem Irrigationsfluidbehälter (2) zu einem ophthalmochirurgischen Handstück (6) für eine Phakoemulsifikation strömen kann,
- eine erste Fluidpumpe (5), welche in Strömungsrichtung zwischen dem Irrigationsfluidbehälter (2) und dem Handstück (6) angeordnet ist und eingerichtet ist, Irrigationsfluid (4) zum Handstück (6) zu fördern,
- eine erste Volumenstrom-Bestimmungsvorrichtung (13), welche in Strömungsrichtung zwischen der ersten Fluidpumpe (5) und dem Handstück (6) angeordnet ist und eingerichtet ist, einen ersten Volumenstrom (Q1) zu bestimmen,
- eine Aspirationsfluidleitung (10), durch welche Aspirationsfluid von dem Handstück (6) zu einem Sammelbehälter (12) strömen kann,
- eine zweite Fluidpumpe (11), welche in Strömungsrichtung zwischen dem Handstück (6) und dem Sammelbehälter (12) angeordnet ist und eingerichtet ist, Aspirationsfluid zum Sammelbehälter (12) zu fördern,
- eine zweite Volumenstrom-Bestimmungsvorrichtung (14), welche in Strömungsrichtung zwischen dem Handstück (6) und der zweiten Fluidpumpe (11) angeordnet ist und eingerichtet ist, einen zweiten Volumenstrom (Q2) zu bestimmen,
**gekennzeichnet durch**
- ein Differenzelement (15), welches mit der ersten Volumenstrom-Bestimmungsvorrichtung (13) und der zweiten Volumenstrom-Bestimmungsvorrichtung (14) gekoppelt und eingerichtet ist, eine Differenz aus dem ersten Volumenstrom (Q1) und dem zweiten Volumenstrom (Q2) zu bilden, um ein Differenz-Volumenstrom-Signal (16) zu bilden,
- eine Steuerungsvorrichtung (17) mit einem ersten Eingang (171) zum Empfangen des Differenz-Volumenstrom-Signals (16) und einem zweiten Eingang (172) zum Empfangen eines Signals (21) für einen Irrigationsfluid-Solldruck, wobei die Steuerungseinrichtung (17) eingerichtet ist, aus dem Differenz-Volumenstrom-Signal (16) und dem Irrigationsfluid-Solldruck-Signal (21) ein Signal (22) für einen Irrigationsfluid-Steuerdruck zu ermitteln und dieses an einem Ausgang der Steuerungsvorrichtung (17) der ersten Fluidpumpe (5) zuzuführen.

2. Ophthalmochirurgische Einrichtung (1) nach Anspruch 1, wobei die Steuerungsvorrichtung (17) aufweist:
ein Multiplikationselement (18), welches eingerichtet ist, an einem Eingang das Differenz-Volumenstrom-Signal (16) zu empfangen und an einem Ausgang ein Signal (19) für einen Differenzdruck auszugeben, und
ein Addierelement (20), welches eingerichtet ist, an einem ersten Eingang das Signal (21) für den Irrigationsfluid-Solldruck zu empfangen, und an einem zweiten Eingang das Signal für den Differenzdruck (19) zu empfangen, und an einem Ausgang das Signal (22) für den Irrigationsfluid-Steuerdruck dem Ausgang der Steuerungseinrichtung (17) zuzuführen.

3. Ophthalmochirurgische Einrichtung (1) nach einem der vorherigen Ansprüche, wobei ein erstes Zeitglied (23) vorgesehen ist, welches eingerichtet ist, den Verlauf des Irrigationsfluid-Solldruckes in Abhängigkeit von der Zeit vorzugeben, und mit dem zweiten Eingang (172) der Steuerungsvorrichtung (17) gekoppelt ist.

4. Ophthalmochirurgische Einrichtung (1) nach einem der vorherigen Ansprüche, wobei ein zweites Zeitglied (25) vorgesehen ist, welches eingerichtet ist, den Aspirationsfluid-Solldruck in Abhängigkeit von der Zeit vorzugeben und der zweiten Fluidpumpe (11) zuzuführen.

5. Ophthalmochirurgische Einrichtung (1) nach einem der Ansprüche 3 oder 4, wobei eine erste Zeitkonstante des ersten Zeitgliedes (23) einstellbar ist.

6. Ophthalmochirurgische Einrichtung (1) nach Anspruch 5, wobei die erste Zeitkonstante in einem Bereich von 50 Millisekunden bis 1000 Millisekunden liegt.

7. Ophthalmochirurgische Einrichtung (1) nach einem der Ansprüche 4 bis 6, wobei eine zweite Zeitkonstante des zweiten Zeitgliedes (25) einstellbar ist.

8. Ophthalmochirurgische Einrichtung (1) nach Anspruch 7, wobei die zweite Zeitkonstante in einem Bereich von 50 Millisekunden bis 1000 Millisekunden liegt.

9. Ophthalmochirurgische Einrichtung (1) nach einem der Ansprüche 7 oder 8, wobei eine Differenz zwischen der ersten Zeitkonstante und der zweiten Zeitkonstante einen Betrag aufweist, welcher in einem Bereich von größer als null und kleiner als 200 Millisekunden liegt.

## Claims

1. Ophthalmic surgical instrument (1), having:
- an irrigation fluid line (4) through which irrigation fluid (3) can flow from an irrigation fluid container (2) to an ophthalmic surgical handpiece (6) for phacoemulsification,
- a first fluid pump (5) arranged, in a direction of flow, between the irrigation fluid container (2) and the handpiece (6) and configured to convey irrigation fluid (4) to the handpiece (6),
- a first volumetric flow determination device (13) arranged, in the direction of flow, between the first fluid pump (5) and the handpiece (6) and configured to determine a first volumetric flow (Q1),
- an aspiration fluid line (10) through which aspiration fluid can flow from the handpiece (6) to a collecting container (12),
- a second fluid pump (11) arranged, in the direction of flow, between the handpiece (6) and the collecting container (12) and configured to convey aspiration fluid to the collecting container (12),
- a second volumetric flow determination device (14) arranged, in the direction of flow, between the handpiece (6) and the second fluid pump (11) and configured to determine a second volumetric flow (Q2),
**characterized by**
- a difference element (15) coupled to the first volumetric flow determination device (13) and the second volumetric flow determination device (14) and configured to form a difference from the first volumetric flow (Q1) and the second volumetric flow (Q2), in order to form a differential volumetric flow signal (16),
- a control device (17) with a first input (171) for receiving the differential volumetric flow signal (16) and with a second input (172) for receiving a signal (21) for an irrigation fluid setpoint pressure, wherein the control device (17) is configured to determine, from the differential volumetric flow signal (16) and the irrigation fluid setpoint pressure signal (21), a signal (22) for an irrigation fluid control pressure and to supply this signal at an output of the control device (17) to the first fluid pump (5).

2. Ophthalmic surgical instrument (1) according to Claim 1, wherein the control device (17) has:
a multiplication element (18) configured to receive the differential volumetric flow signal (16) at an input and to output a signal (19) for a differential pressure at an output, and
an addition element (20) configured to receive the signal (21) for the irrigation fluid setpoint pressure at a first input and to receive the signal for the differential pressure (19) at a second input and, at an output, to supply the signal (22) for the irrigation fluid control pressure to the output of the control device (17).

3. Ophthalmic surgical instrument (1) according to either of the preceding claims, wherein a first timing element (23) is provided which is configured to predefine the profile of the irrigation fluid setpoint pressure as a function of time and which is coupled to the second input (172) of the control device (17).

4. Ophthalmic surgical instrument (1) according to one of the preceding claims, wherein a second timing element (25) is provided which is configured to predefine the aspiration fluid setpoint pressure as a function of time and to supply it to the second fluid pump (11).

5. Ophthalmic surgical instrument (1) according to either of Claims 3 and 4, wherein a first time constant of the first timing element (23) is adjustable.

6. Ophthalmic surgical instrument (1) according to Claim 5, wherein the first time constant lies in a range of 50 milliseconds to 1000 milliseconds.

7. Ophthalmic surgical instrument (1) according to one of Claims 4 to 6, wherein a second time constant of the second timing element (25) is adjustable.

8. Ophthalmic surgical instrument (1) according to Claim 7, wherein the second time constant lies in a range of 50 milliseconds to 1000 milliseconds.

9. Ophthalmic surgical instrument (1) according to either of Claims 7 and 8, wherein a difference between the first time constant and the second time constant has a quantity that lies in a range of greater than zero and less than 200 milliseconds.

## Revendications

1. Dispositif chirurgical ophtalmique (1), possédant :
- une conduite à fluide d'irrigation (4), à travers laquelle un fluide d'irrigation (3) peut s'écouler d'un réservoir à fluide d'irrigation (2) à une pièce à main de chirurgie ophtalmique (6) pour une phacoémulsification,
- une première pompe à fluide (5), qui est disposée dans le sens de l'écoulement entre le réservoir à fluide d'irrigation (2) et la pièce à main (6) et qui est conçue pour transporter le fluide d'irrigation (4) vers la pièce à main (6),
- un premier arrangement de détermination de débit volumique (13), qui est disposé dans le sens de l'écoulement entre la première pompe à fluide (5) et la pièce à main (6) et qui est conçu pour déterminer un premier débit volumique (Q1),
- une conduite à fluide d'aspiration (10), à travers laquelle un fluide d'aspiration peut s'écouler de la pièce à main (6) à un récipient collecteur (12),
- une deuxième pompe à fluide (11), qui est disposée dans le sens de l'écoulement entre la pièce à main (6) et le récipient collecteur (12) et qui est conçue pour transporter le fluide d'aspiration vers le récipient collecteur (12),
- un deuxième arrangement de détermination de débit volumique (14), qui est disposé dans le sens de l'écoulement entre la pièce à main (6) et la deuxième pompe à fluide (11) et qui est conçu pour déterminer un deuxième débit volumique (Q2),
**caractérisé par**
- un élément de différence (15), qui est connecté au premier arrangement de détermination de débit volumique (13) et au deuxième arrangement de détermination de débit volumique (14) et conçu pour former une différence à partir du premier débit volumique (Q1) et du deuxième débit volumique (Q2) afin de former un signal de débit volumique différentiel (16),
- un arrangement de commande (17) comprenant une première entrée (171), destinée à recevoir le signal de débit volumique différentiel (16), et une deuxième entrée (172), destiné à recevoir un signal (21) pour une pression de consigne de fluide d'irrigation, l'arrangement de commande (17) étant conçu pour déterminer, à partir du signal de débit volumique différentiel (16) et du signal de pression de consigne de fluide d'irrigation (21), un signal (22) pour une pression de commande de fluide d'irrigation et acheminer celui-ci à une sortie de l'arrangement de commande (17) de la première pompe à fluide (5).

2. Dispositif chirurgical ophtalmique (1) selon la revendication 1, l'arrangement de commande (17) possédant :
un élément de multiplication (18), lequel est conçu pour recevoir le signal de débit volumique différentiel (16) au niveau d'une entrée et délivrer un signal (19) pour une pression différentielle au niveau d'une sortie, et
un élément additionneur (20), lequel est conçu pour recevoir le signal (21) pour la pression de consigne de fluide d'irrigation au niveau d'une première entrée, et recevoir le signal pour la pression différentielle (19) au niveau d'une deuxième entrée et amener au niveau d'une sortie le signal (22) pour la pression de commande de fluide d'irrigation à la sortie de l'arrangement de commande (17).

3. Dispositif chirurgical ophtalmique (1) selon l'une des revendications précédentes, un premier organe temporisateur (23) étant présent, lequel est conçu pour prédéfinir le tracé de la pression de consigne de fluide d'irrigation en fonction du temps et est connecté à la deuxième entrée (172) de l'arrangement de commande (17).

4. Dispositif chirurgical ophtalmique (1) selon l'une des revendications précédentes, un deuxième organe temporisateur (25) étant présent, lequel est conçu pour prédéfinir la pression de consigne de fluide d'aspiration en fonction du temps et l'acheminer à la deuxième pompe à fluide (11).

5. Dispositif chirurgical ophtalmique (1) selon l'une des revendications 3 ou 4, une première constante de temps du premier organe temporisateur (23) étant réglable.

6. Dispositif chirurgical ophtalmique (1) selon la revendication 5, la première constante de temps étant comprise dans une plage de 50 millisecondes à 1000 millisecondes.

7. Dispositif chirurgical ophtalmique (1) selon l'une des revendications 4 à 6, une deuxième constante de temps du deuxième organe temporisateur (25) étant réglable.

8. Dispositif chirurgical ophtalmique (1) selon la revendication 7, la deuxième constante de temps étant comprise dans une plage de 50 millisecondes à 1000 millisecondes.

9. Dispositif chirurgical ophtalmique (1) selon l'une des revendications 7 ou 8, une différence entre la première constante de temps et la deuxième constante de temps présentant un montant qui est compris dans une plage entre plus que zéro et moins que 200 millisecondes.
